# FASCICULE DE BREVET EUROPEEN

(11) **EP 1 771 498 B1**
(45) Date de publication et mention de la délivrance du brevet: **18.11.2009**
(21) Numéro de dépôt: 05791929.2
(22) Date de dépôt: 19.07.2005
(51) Int. Cl.: C08G 83/00

(54) **POLYAMINOACIDES BRANCHES, FONCTIONNALISES PAR DES GROUPEMENTS HYDROPHOBES ET LEURS APPLICATIONS NOTAMMENT THERAPEUTIQUES**
MITTELS WASSERABWEISENDER GRUPPEN FUNKTIONALISIERTE VERZWEIGTE POLYAMINOSÄUREN UND DEREN VERWENDUNG, INSBESONDERE FÜR THERAPEUTISCHE ZWECKE
BRANCHED POLYAMINOACIDS, FUNCTIONALIZED BY HYDROPHOBIC GROUPS AND USES THEREOF IN PARTICULAR IN THERAPY

(30) Priorité: 30.07.2004 FR 0408477
(43) Date de publication de la demande: 11.04.2007
(73) Titulaire: FLAMEL TECHNOLOGIES, 69200 Vénissieux (FR)
(72) Inventeur: SOULA, Rémi, F-69007 LYON (FR); POULIQUEN, Gauthier, F-69007 LYON (FR); CHAN, You-Ping, F-69008 LYON (FR)
(74) Mandataire: Fleurance, Raphaël
(86) Numéro de dépôt international: PCT/FR2005/050594
(87) Numéro de publication internationale: WO 2006/021706

(56) Documents cités:
- WO-A-2004/060968
- FR-A- 2 840 614
- FR-A- 2 843 117

## Description

La présente invention concerne des nouveaux matériaux à base de polyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant, de préférence, sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intramusculaire, intradermique, intrapéritonéale, intracérébrale, buccale, etc.

Les PA plus particulièrement, mais non limitativement, concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôler leur vitesse de libération afin de maintenir un niveau thérapeutique sur une durée définie,
- et/ou de les véhiculer (en les protégeant) au site d'action.

A ces fins, plusieurs types de polymères ont été étudiés et certains sont même disponibles commercialement. On peut citer, par exemple, les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou être biodégradables. Sur ce dernier aspect, il est de surcroît essentiel que la biodégradation dans l'organisme génère des produits non-toxiques.

Un autre aspect aussi important dans le développement d'un polymère associatif est sa solubilité dans l'eau. La possibilité de solubiliser une quantité élevée de polymère permet d'avoir un rapport polymère/principe actif adapté au profil de libération souhaité.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US-B-4,652,441 décrit des microcapsules de polylactide encapsulant l'hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (alcool polyvinylique). La libération du PA peut se faire sur une période de plus de deux semaines après injection sous-cutanée.

Le brevet US-B-6,153,193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US-B-4,351,337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US-B-4,888,398 décrit des polymères à base de polyglutamate ou polyaspartate, et éventuellement polyleucine, avec des groupements pendants de type alkyloxycarbonylméthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US-B-5,904,936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Le brevet US-B-5,449,513 décrit des copolymères bloc amphiphiles comprenant un bloc polyoxyéthylène et un bloc polyaminoacide, par exemple poly(béta-benzyl-L-aspartate). Ces polymères polyoxyéthylène-polybenzylaspartate forment des micelles qui sont aptes à encapsuler des molécules actives hydrophobes telles que l'adriamycine ou l'indométhacine.

La demande de brevet WO-A-99/61512 décrit des polylysines et des polyornithines fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle forment, en présence de cholestérol, des vésicules capables d'encapsuler la doxorubicine ou l'ADN. Ces polymères à base de polylysines sont cationiques en milieu physiologique.

Le brevet US-B-6,630,171, de la demanderesse, décrit des polymères blocs ou aléatoires poly(glutamate de sodium)-poly(glutamate de méthyle, d'éthyle, d'hexadécyle ou de dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période. Ces copolyaminoacides linéaires amphiphiles sont modifiés par la présence d'une chaîne latérale alkyle hydrophobe. Ces groupements alkyles sont greffés de façon covalente sur le polymère via une fonction ester. Ces polymères sont anioniques en milieu physiologique.

Dans le même domaine, la demanderesse a décrit dans plusieurs demandes de brevets, des polymères à base de polyglutamate avec des concepts apparentés.
La demande WO-A-03/104303 décrit des polyaminoacides anioniques fonctionnalisés par de l'alpha-tocopherol. La demande WO-A-04/013206 décrit des polyaminoacides anioniques comportant des groupements hydrophobes et caractérisés en ce que ces groupements sont reliés au polymère par l'intermédiaire d'une rotule contenant deux fonctions amides, et plus précisément via un espaceur de type lysine ou ornithine.

La demande PCT/FR03/03458 non publiée décrit des polyaminoacides fonctionnalisés par au moins un groupement oligoaminoacide à base de leucine et/ou isoleucine et/ou valine et/ou phenylalanine.

La demande de brevet WO-A-87/03891 décrit des polymères amphiphiles, linéaires, branchés ou en étoile, avec au moins deux groupements hydrophobes liés seulement à leurs extrémités. Cette demande de brevet concerne essentiellement des polymères hydrophiles neutres à base de polyéthylène glycol, comme en témoigne tous les exemples de ce brevet. Or, ce type de polymère n'est pas biodégradable, ce qui constitue un inconvénient majeur.

Ainsi, même si de très nombreuses solutions techniques sont développées et proposées dans l'art antérieur pour la vectorisation des principes actifs médicamenteux, la réponse à l'ensemble des exigences est difficile à obtenir et demeure non satisfaisante. Plus spécifiquement, on a pu identifier un besoin non satisfait en un matériau biodégradable pour la réalisation de particules de vectorisation de principes actifs, ce matériau devant être capable de former une suspension aqueuse de nano- ou microparticules de vectorisation propres à s'associer réversiblement à des principes actifs et dans lequel l'une des améliorations recherchées serait d'avoir un rapport polymère/principe actif le plus élevé possible.

Dans ce contexte, l'un des objectifs essentiels de la présente invention est de fournir de nouveaux polyaminoacides branchés, amphiphiles et anioniques à pH physiologique animal (par exemple de l'ordre de 7,4), qui représentent un perfectionnement par rapport à ceux décrits dans les brevets ou demandes de brevets US-B-6,630,171, WO-A-03/104303, WO-A-04/013206 et la demande PCT/FR03/03458 non publiée notamment en termes de formulation d'un principe actif tel qu'une protéine thérapeutique..

Un autre objectif essentiel de la présente invention est que ces polymères soient aptes à être utilisés pour la vectorisation de PA et permettent de satisfaire de manière optimale à toutes les spécifications du cahier des charges, à savoir notamment :
o capacité :
   ■ à former aisément et économiquement des suspensions colloïdales aqueuses stables,
   ■ à s'associer facilement avec de nombreux principes actifs,
   ■ et à libérer ces principes actifs in vivo,
o biocompatibilité,
o biodégradabilité,
o stabilité à l'hydrolyse.

Cet objectif, parmi d'autres, est atteint par la présente invention qui concerne tout d'abord un polyaminoacide comprenant des unités aspartiques et/ou des unités glutamiques, dont certaines sont porteuses d'un ou de plusieurs groupements hydrophobes (GH), identiques ou différents entre eux,
caractérisé en ce qu'il comprend au moins un type de chaîne principale Cp polyaminoacide comportant des unités aspartiques et/ou des unités glutamiques et présentant une ou plusieurs branches B polyaminoacide, identiques ou différentes entre elles, comportant des unités aspartiques et/ou des unités glutamiques.

Suivant une caractéristique particulièrement préférée du polyaminoacide branché selon l'invention, les greffons hydrophobes sont liés :
→ (I) à la chaîne principale Cp seulement,
→ (II) à la chaîne principale Cp et à la/aux branches B,
→ (III) ou à la/aux branches B seulement.

Il est du mérite de la demanderesse d'avoir mis au point un polyaminoacide branché, amphiphile et porteur de GH identiques ou différents entre eux, répartis de manière aléatoire dans la structure branchée.

Le polyaminoacide concerné peut être un homopolymère (poly[Glu] ou poly[Asp]) ou un copolymère (poly[Glu]-poly[Asp]).

Les trois familles (I), (II), (III) préférées de polyaminoacide branché selon l'invention peuvent être symbolisées par les structures schématiques suivantes :

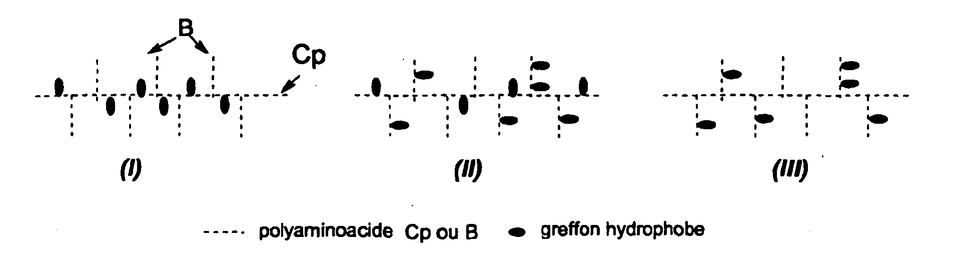

Les structures des familles (I), (II), (III) se distinguent par les positions de greffage des groupements hydrophobes :
- dans la structure (I), les greffons sont situés de façon aléatoire uniquement sur la chaîne Cp (ou squelette) ;
- dans la structure (II), les greffons sont situés de façon aléatoire à la fois sur la chaîne Cp (ou squelette) et sur les branches B ;
- dans la structure (III), les greffons sont situés de façon aléatoire uniquement sur les branches B.

Pour obtenir les familles préférées (I), (II), (III); la demanderesse a eu le mérite de combiner, de façon tout à fait judicieuse et avantageuse, des polyaminoacides (de préférence des homopolyaminoacides) particuliers branchés, biodégradables (e.g. polyAsp ou polyGlu), avec des groupements hydrophobes GH.

Ces nouveaux polymères amphiphiles branchés se sont avérés être particulièrement bien adaptés pour la vectorisation des protéines.

Au sens de l'invention:
➢ le terme *"polyaminoacide* "couvre, d'une part les PAA comportant un seul type d'unité "acide aminé" (par exemple soit des unités Glu (glutamiques ou glutamates), soit des unités Asp (aspartiques ou aspartates), soit un copolyaminoacide (comportant un mélange d'unités d'acides amines Glu et Asp dans un enchainement de type aléatoire, gradient ou bloc) et, d'autre part, aussi bien les oligoaminoacides comprenant de 2 à 20 unités "acide aminé" que les polyaminoacides comprenant plus de 20 unités "acide aminé";
➢ le terme *"unité acide aminé"* visé un motif monomérique ou non formé par un squelette d'un acide aminé donné, quelles que puissent être les substitutions pour autant qu'elles ne modifient pas la nature de l'acide aminé concerné.

Ces polymères présentent des propriétés surprenantes de fluidité, d'association et/ou d'encapsulation avec un ou plusieurs principes actifs, en comparaison avec des produits analogues.
De plus, ils sont facilement dégradés, en présence d'enzymes, en catabolites/métabolites non toxiques (acides aminés).

Au sens de l'invention et dans tout le présent exposé, les termes "association" ou "associer" employés pour qualifier les relations entre un ou plusieurs principes actifs et les homopolyaminoacides, signifient en particulier que le ou les principes actifs sont liés au(x) homopolyaminoacide(s) notamment par une liaison faible, par exemple par liaison ionique et/ou par contact hydrophobe, et/ou sont encapsulés par le ou les homopolyaminoacides.

Avantageusement, au moins l'un des groupements hydrophobes GH est inclus dans un greffon hydrophobe comprenant au moins une rotule (ou motif) d'espacement ("spacer") permettant de relier le groupement hydrophobe GH à une chaîne de polyaminoacide (par exemple une chaîne principale -squelette-polyaminoacide). Cette rotule peut comprendre, e.g. au moins une liaison covalente directe et/ou au moins une liaison amide et/ou au moins une liaison ester. Par exemple, la rotule peut être du type de celles appartenant au groupe comportant : les unités "acide aminé" différentes de l'unité monomérique constitutive du polyaminoacide, les dérivés des aminoalcools, les dérives des diamines, les dérivés des diols et les dérivés des hydroxyacides.

Le greffage des GH sur la chaîne polyaminoacide peut passer par la mise en oeuvre de précurseurs de GH, aptes à se lier à la chaîne polyaminoacide.

Les précurseurs des GH sont, en pratique et sans que cela ne soit limitatif, choisis dans le groupe comprenant les alcools et les amines, ces composés pouvant être fonctionnalisés facilement par l'homme de l'art. Le greffage des GH est explicité plus en détail ci-après dans la description du procédé d'obtention des polyaminoacides selon l'invention.

Suivant une caractéristique préférée, le groupement hydrophobe GH du greffon hydrophobe comporte de 8 à 30 atomes de carbone.

Ces groupements hydrophobes GH sont avantageusement et judicieusement sélectionnés dans le groupe comprenant :
■ les alkyles linéaires ou ramifiés en C8 à C30 pouvant comporter éventuellement au moins une insaturation et/ou au moins un hétéroatome,
■ les alkylaryles ou arylalkyles en C8 à C30 pouvant comporter éventuellement au moins une insaturation et/ou au moins un hétéroatome,
■ et les (poly)cycliques en C8 à C30 pouvant comporter éventuellement au moins une insaturation et/ou au moins un hétéroatome.

Suivant une caractéristique préférée de l'invention, au moins un greffon hydrophobe, de préférence le/les groupements hydrophobes GH dudit greffon, présente au moins une charge anionique et/ou une ou plusieurs fonctions ionisables identiques ou différentes entre elles et capables de donner chacune naissance à au moins une charge anionique.

Avantageusement, le caractère anionique ou anionisable de tout ou partie des greffons hydrophobes est donné par le ou les GH. D'où il s'ensuit que tout ou partie des groupements hydrophobes GH des greffons hydrophobes présente chacun au moins une charge anionique et/ou au moins une fonction ionisable capable de donner naissance à au moins une charge anionique.

De préférence, la fonction ionisable du GH capable de donner naissance à au moins une charge anionique est choisie dans le groupe de fonctions comprenant : la fonction carboxylique/carboxylate, la fonction sulfonique/sulfonate, la fonction sulfurique/sulfate et la fonction phosphorique/phosphate.

Lorsqu'ils ne sont pas ionisés ou ionisables, les groupements hydrophobes GH peuvent être, par exemple, dérivés de groupements choisis dans le groupe comprenant : l'octanol, le dodécanol, le tétradécanol, l'héxadécanol, l'octadécanol, l'oleylalcool, le tocophérol, le cholestérol ou l'acide lithocholique.

De préférence, les (homo)polyaminoacides selon la présente invention sont des homooligomères ou des homopolymères comprenant des unités d'alpha-L-glutamate et/ou d'alpha-L-glutamique ou des unités d'alpha-L-aspartate et/ou d'alpha-L-aspartique.

De manière plus préférée encore, les polyaminoacides selon l'invention sont des polyglutamates qui répondent aux formules générales (**I**), (**II**) et (**III**) suivantes : 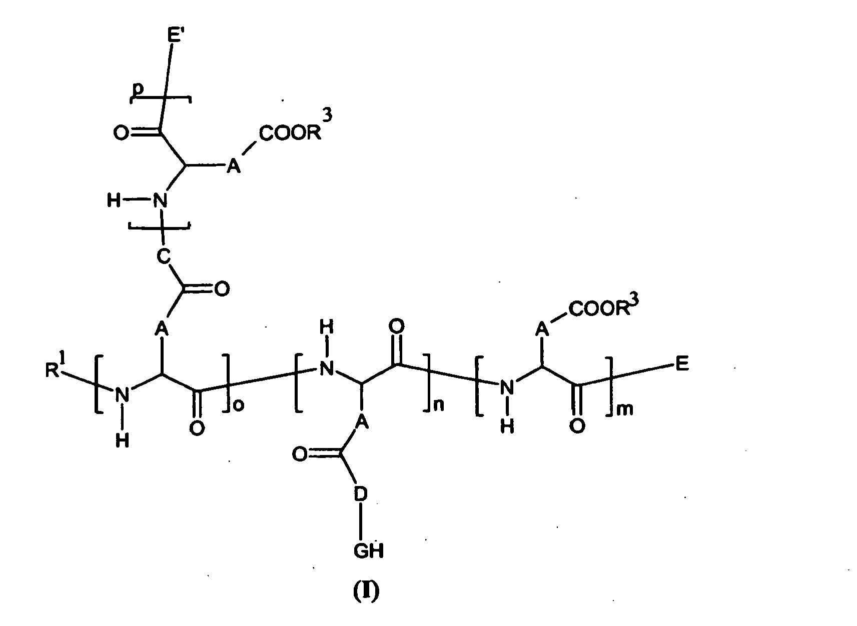 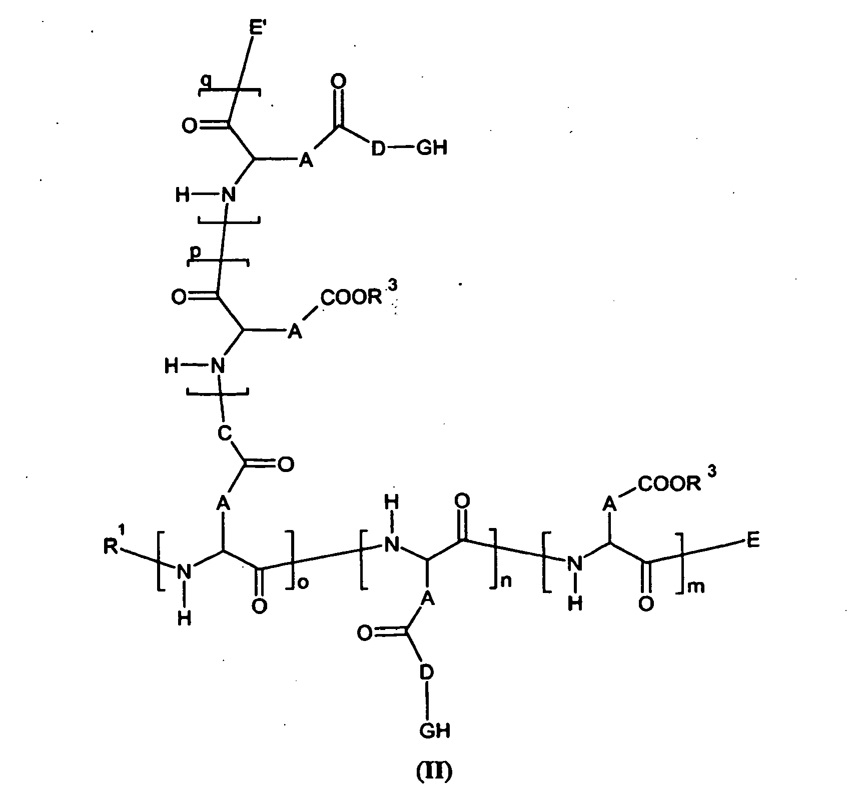 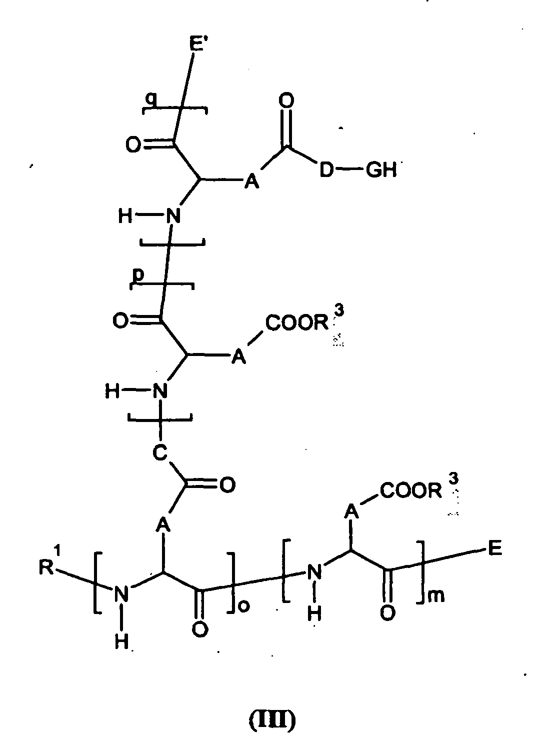 dans lesquelles :
■ A représente indépendamment un -CH₂- (unité aspartique) ou -CH₂-CH₂-(unité glutamique) ;
■ R¹ représente un H, un groupe acyle linéaire en C2 à C10 ou ramifié en C3 à C10, ou un pyroglutamate ;
■ E, E' représentent indépendamment :
   - un OR³, R³ répondant à la définition donnée ci-dessous,
   - un groupement NHR² dans laquelle R² représente un H, un alkyle linéaire en C2 à C 10 ou ramifié en C3 à C 10 ou un benzyle,
   - une unité acide aminé terminale liée par l'azote et dont la fonction(s) acide(s) est éventuellement modifiée par une amine ou un alcool répondant aux définitions NHR² et OR² respectivement;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium ;
   - les cations organiques avantageusement choisis dans le sous-groupe comprenant :
      • les cations à base d'amine,
      • les cations à base d'oligoamine,
      • les cations à base de polyamine (1a polyéthylèneimine étant particulièrement préférée),
      • les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine;
■ C représente une liaison directe ou un groupement de liaison choisi parmi un reste d'acide aminé (de préférence naturel) ou un hydroxyalcool comportant de 1 à 6 atomes de carbones;
■ les groupements D-GH représentent chacun indépendamment les uns des autres un radical dans lequel :
   - D est un groupement de liaison -O-, -NH-, -N-alkyle- (C1 à C5), un résidu d'acide aminé (de préférence naturel), un diol, une diamine, un aminoalcool ou un hydroxyacide comportant de 1 à 6 atomes de carbone, et
   - GH représente un groupement hydrophobe comportant 8 à 30 atomes de carbone;
      • alkyles linéaires ou ramifiés en C8 à C30 pouvant comporter éventuellement au moins une insaturation et/ou au moins un hétéroatome (de préférence O et/ou N et/ou S), ou
      • alkylaryles ou arylalkyle en C8 à C30 pouvant comporter éventuellement au moins une insaturation et/ou au moins un hétéroatome (de préférence O et/ou N et/ou S), ou
      • (poly)cycliques en C8 à C30 pouvant comporter éventuellement au moins une insaturation et/ou au moins un hétéroatome (de préférence O et/ou N et/ou S).
■ (n+q)/(q+p+o+n+m) est défini comme le taux de greffage molaire des groupements hydrophobes GH et varie de 0,5 à 90 % molaire ;
■ q+p+o+n+m varie de 20 à 5000, de préférence entre 30 et 2000 ;
■ o+n+m (le squelette) varie de 10 à 500, de préférence entre 30 et 300 ;
■o/(o+n+m) est défini comme le taux de greffage des branches et varie entre 1 et 50 % molaire ;
■ la chaîne principale Cp de ces polyaminoacides (I) (II) (III) est constituée par les motifs récurrents -[ ]ₒ-[ ]ₙ-[ ]ₘ- ou -[ ]ₒ-[ ]ₘ- et leurs branches B par les motifs récurrents -[ ]p- ou -[ ]ₚ-[ ]_{q}-;
■ et les groupements hydrophobes GH et les branches B sont disposés de façon aléatoire.

Selon un premier mode de réalisation de l'invention, la chaîne principale Cp et les branches B des polyaminoacides sont des homopolymères d'alpha-L-glutamate ou d'alpha-L-glutamique.

Selon un deuxième mode de réalisation de l'invention, la chaîne principale Cp et les branches B des polyaminoacides sont des homopolymères d'alpha-L-aspartate ou d'alpha-L-aspartique.

Selon un troisième mode de réalisation de l'invention, la chaîne principale Cp et les branches B des polyaminoacides sont des copolymères d'alpha-L-aspartate/alpha-L-glutamate ou d'alpha-L-aspartique/alpha-L-glutamique.

Avantageusement, la distribution des unités aspartiques et/ou glutamiques de la chaîne polyaminoacide principale est telle que les polymères ainsi constitués sont soit aléatoires, soit de type bloc, soit de type multibloc.

Il est par ailleurs préférable que le taux de greffage molaire en motif hydrophobe des polyaminoacides selon l'invention, soit compris entre 2 et 100 %, et de préférence entre 5 et 50 %.

Selon un autre mode de définition, les polyaminoacides selon l'invention ont une masse molaire qui se situe entre 2 000 et 800 000 g/mole, et de préférence entre 5 000 et 300 000 g/mole.

Selon d'autres variantes, les polyaminoacides selon l'invention peuvent être porteurs d'au moins un greffon de type polyéthylène glycol lié à une unité glutamate et/ou aspartate.

Naturellement, l'invention couvre également des mélanges de polyaminoacides tels que définis ci-dessus.

Par exemple, les polyaminoacides branchés hydrophobes selon l'invention peuvent comprendre différents types de chaînes principales (ou squelettes), qui se distinguent les unes des autres par le nombre d'unités "acide aminé" et/ou par la nature desdites unités. Il en est de même s'agissant des branches B qui peuvent être identiques ou différentes entre elles par le nombre et/ou la nature des unités "acide aminé" qui les composent.

De manière remarquable, les polyaminoacides de l'invention sont susceptibles d'être utilisés de plusieurs façons selon la nature des groupements hydrophobes et le degré de polymérisation du polyaminoacide. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
*"*Microspheres, Microcapsules and Liposomes ; vol 1. Preparation and chemical applications" Ed. R. Arshady, Citus Books 1999. ISBN : 0-9532187-1-6.
*"*Sustained-Release Injectable Products" Ed. J. Senior et M. Radomsky, Interpharm Press 2000. ISBN : 1-57491-101-5.
*"*Colloidal Drug Delivery Systems" Ed. J. Kreuter, Marcel Dekker, Inc. 1994. ISBN : 0-8247-9214-9.
*"*Handbook of Pharmaceutical Controlled Release Technology" Ed. D.L. Wise, Marcel Dekker, Inc. 2000. ISBN : 0-8247-0369-3.

Ces polyaminoacides sont en outre extrêmement intéressants du fait que, selon la longueur de l'homopolymère (degré de polymérisation) et la nature des groupements hydrophobes, ils se dispersent dans l'eau à pH 7,4 (par exemple avec un tampon phosphate) pour donner des solutions ou des suspensions colloïdales ou des gels structurés ou non, en fonction de la concentration en homopolymères. De plus, les polyaminoacides (sous forme de particules ou non), peuvent encapsuler ou s'associer aisément avec des principes actifs tels que des protéines, peptides ou petites molécules. La mise en forme préférée est celle décrite dans le brevet US-B-6,630,171 de la demanderesse et qui consiste à disperser l'homopolymère dans l'eau et à incuber la solution en présence d'un principe actif (PA). Cette solution colloïdale de particules de vectorisation constituées des homopolyaminoacides selon l'invention, peut ensuite être filtrée sous 0,2 µm puis directement injectée à un patient.

Quand le rapport hydrophile/hydrophobe diminue, l'homopolymère peut alors former des microparticules capables d'associer ou d'encapsuler des PA. Dans ce contexte, la mise en forme des microparticules peut se faire en co-solubilisant le PA et l'homopolymère dans un solvant organique approprié puis le mélange précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

Selon une variante, l'homopolymère peut être solubilisé dans un solvant biocompatible tel que la N-méthylpyrrolidone ou une huile appropriée telle que le Mygliol® puis injecté en intramusculaire ou sous-cutanée ou dans une tumeur. La diffusion du solvant ou de l'huile conduit à la précipitation de l'homopolymère sur le site d'injection et forme ainsi un dépôt. Ces dépôts assurent ensuite une libération contrôlée par diffusion et/ou par érosion et/ou par dégradation hydrolytique ou enzymatique de l'homopolymère.

Indépendamment du fait que la forme microparticulaire du polyaminoacide selon l'invention est préférée, les polymères de l'invention, sous forme neutre ou ionisée, sont de façon plus générale, utilisables seuls ou dans une composition liquide, solide ou gel et dans un milieu aqueux ou organique.

Il convient de comprendre que le polymère à base de polyaminoacides contient des fonctions carboxyliques qui sont soit neutres (forme COOH), soit ionisées (anion COO⁻), selon le pH et la composition. Pour cette raison, la solubilité dans une phase aqueuse est directement fonction du taux de COOH libre du polymère (non greffé par le motif hydrophobe) et du pH. En solution aqueuse, le contre-cation peut être un cation métallique tel que le sodium, le calcium ou le magnésium, ou un cation organique tel que la triéthanolamine, la tris(hydroxyméthyl)-aminométhane ou une polyamine tel que la polyéthylèneimine.

Les polymères de l'invention sont par exemple obtenus par des méthodes connues de l'homme de l'art. Tout d'abord, rappelons que pour l'obtention de polyaminoacide de type alpha, la technique la plus courante est basée sur la polymérisation d'anhydrides de N-carboxy-aminoacides (NCA), décrite, par exemple, dans l'article *"*Biopolymers, 1976, 15, 1869 et dans l'ouvrage de H.R. Kricheldorf "alpha-Aminoacid-N-carboxy Anhydrides and related Heterocycles" Springer Verlag (1987). Le dérivé de NCA est de préférence NCA-Glu-O-Bz (Bz = Benzyle), car le groupement benzyle peut être sélectivement hydrolysé sans toucher d'autres fonctions chimiques des homopolymères ou du groupement hydrophobe.

Un certain nombre de polymères utilisables selon l'invention, par exemple, de type poly(alpha-L-aspartique), poly(alpha-L-glutamique), poly(alpha-D-glutamique) et poly(gamma-L-glutamique) de masses variables sont disponibles commercialement. Le polymère polyaspartique de type alpha-bêta est obtenu par condensation de l'acide aspartique (pour obtenir un polysuccinimide) suivie d'une hydrolyse basique (cf. Tomida et al. Polymer 1997, 38, 4733-36).

Les étapes ci-dessous illustrent les synthèses des polymères préférés de l'invention : Polyglutamates de structures (I), (II) et (III). Le mode d'obtention de ces polymères n'est pas limitatif. Quelques schémas de synthèse sont donnés ci-dessous à titre d'illustration. La chimie de polymérisation et de réaction de couplage des groupements est classique et bien connue de l'homme de l'art (voir par exemples les brevets ou demandes de brevets de la demanderesse cités précédemment).

La synthèse d'un polymère de type I peut se faire selon les étapes suivantes :
1. synthèse du squelette : polyglutamique avec un rapport molaire initiateur/monomère NCA de 1/(o+n+m). (Cette polymérisation est suivie d'une hydrolyse comme décrit dans la demande de brevet FR-A-2 801 226. Le polymère est isolé sous sa forme polyacide ;
2. greffage des greffons hydrophobes à groupements GH sur le polymère en utilisant un rapport molaire n/(o+n+m) selon une méthode décrit dans la demande WO-A-03/104303 de la demanderesse ;
3. synthèse des branches : idem précédente avec un rapport molaire initiateur/monomère NCA de 1/(p+q), par exemple utilisant un NCA de glutamate de benzyle ;
4. greffage des branches sur le polymère en utilisant un rapport molaire o/(o+n+m) ;
5. hydrolyse des groupements benzyles pour obtenir le polymère souhaité.

La synthèse d'un polymère de type (II) peut se faire de façon analogue en synthétisant d'abord le polymère branché sans greffon hydrophobe à groupement GH et la réaction de greffage des greffons hydrophobes à groupements GH est réalisée en dernier (étape 2). Celle du polymère de type (III) peut se faire en greffant d'abord les greffons hydrophobes à groupements GH sur les branches et le greffage des branches se fait dans la dernière étape.

Ces méthodes seront mieux comprises à travers la description des exemples.

Il doit être observé que le degré de polymérisation est défini par le rapport molaire de l'initiateur sur le monomère.

Le couplage du greffon hydrophobe à GH avec une fonction acide du polymère est réalisé aisément par réaction du polyaminoacide en présence d'un carbodiimide comme agent de couplage et optionnellement, un catalyseur tel que le 4-diméthylaminopyridine et dans un solvant approprié tel que la diméthylformamide (DMF), la N-méthyl pyrrolidone (NMP) ou la diméthylsulfoxide (DMSO). Le carbodiimide est par exemple, le dicyclohexylcarbodiimide ou le diisopropylcarbodiimide. D'autres réactifs de couplages tels que les chloroformates peuvent également être utilisés (voir par exemple l'ouvrage de Bodanszky « Principles of Peptide Synthesis » Springer Verlag 1984 pour des exemples d'agents de couplages). Le taux de greffage est contrôlé chimiquement par la stoechiométrie des constituants et réactifs ou le temps de réaction. Les greffons hydrophobes ou les branches fonctionnalisés par un acide aminé autre celui du polymère sont obtenus par couplage peptidique classique ou par condensation directe par catalyse acide. Ces techniques sont bien connues de l'homme de l'art.

Selon un autre de ses aspects, l'invention vise une composition pharmaceutique, cosmétique, diététique ou phytosanitaire comprenant au moins un polyaminoacide tel que défini ci-dessus et éventuellement au moins un principe actif, qui peut être thérapeutique, cosmétique, diététique ou phytosanitaire.

Suivant une disposition intéressante de l'invention, le principe actif est associé au(x) polyaminoacide(s) par une ou plusieurs liaisons autre(s) qu'une (ou que des) liaison(s) chimique(s) covalente(s).
Les techniques d'association d'un ou de plusieurs PA aux polyaminoacides greffés selon l'invention, sont décrites notamment dans le brevet US-B-6,630,171. Elles consistent à incorporer au moins un principe actif dans le milieu liquide contenant des Particules de Vectorisation (PV), de manière à obtenir une suspension colloïdale de PV chargées en ou associées avec un ou plusieurs principe(s) actif(s) PA. Cette incorporation, qui conduit à un piégeage de PA par les PV, peut être réalisée de la manière suivante :
- mise en solution aqueuse de PA, puis ajout des PV, soit sous forme de suspension colloïdale, soit sous forme de PV isolées (lyophilisat ou précipité) ;
- ou ajout de PA, soit en solution, soit à l'état pur ou préformulé, à une suspension colloïdale de particules PV, éventuellement préparée extemporanément par la dispersion de PV sèches dans un solvant approprié, tel que l'eau.

De préférence, le principe actif est une protéine, une glycoprotéine, une protéine liée à une ou plusieurs chaînes polyalkylèneglycol (de préférence PolyEthylèneGlycol (PEG) : "protéine-PEGylée"), un polysaccharide, un liposaccharide, un oligonucléotide, un polynucléotide ou un peptide.

Selon une variante, le principe actif est une "petite" molécule organique hydrophobe, hydrophile ou amphiphile.
Au sens du présent exposé, une "petite" molécule est notamment une petite molécule non protéinique.

Comme exemples de PA susceptibles d'être associés aux polyaminoacides selon l'invention, qu'ils soient ou non sous forme de (nano ou micro)particules, on peut citer :
o les protéines telles que l'insuline, les interférons, les hormones de croissance, les interleukines, l'érythropoïétine ou les cytokines ;
o les peptides telles que la leuprolide ou la cyclosporine ;
o les petites molécules telles que celles appartenant à la famille des anthracyclines, des taxoïdes ou des camptothécines ;
o et leurs mélanges.

Selon un mode de réalisation, la composition de l'invention est sous forme d'un gel, d'une solution, d'une suspension, d'une émulsion, de micelles, de nanoparticules, de microparticules, d'un implant, d'une poudre ou d'un film.

Suivant l'une de ses formes particulièrement préférées, la composition, chargée ou non en principe actif(s), est une suspension colloïdale stable de nanoparticules et/ou de microparticules et/ou de micelles polyaminoacides, dans une phase aqueuse.

Selon une autre mode de réalisation, la composition de l'invention est sous forme de solution dans un solvant biocompatible et peut être injectée par voie sous-cutanée, intramusculaire ou dans une tumeur.

La composition selon l'invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Selon une autre mode de réalisation, la composition peut éventuellement contenir un excipient pour l'ajustement du pH et/ou de l'osmolarité et/ou pour améliorer la stabilité (anti-oxydants) et/ou comme agent anti-microbiens. Ces excipients sont bien connus de l'homme de l'art (se référer à l'ouvrage : Injectable Drug Development, P.K. Gupta et al. Interpharm Press, Denver, Colorado 1999).

Selon une autre variante, la composition selon l'invention est formulée de telle sorte qu'elle soit apte à former un dépôt sur le site d'injection.

L'invention vise aussi des compositions qui comprennent des polyaminoacides selon l'invention et des principes actifs et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles ;
- et/ou des nutriments;
- et/ou de produits cosmétiques ou phytosanitaires.

Selon encore un autre de ses aspects, l'invention vise un procédé de préparation :
- de médicaments, en particulier pour administration orale, nasale, vaginale, oculaire, sous-cutanée, intraveineuse, intramusculaire, intradermique, intrapérito-néale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des liposaccharides, des oligonucléotides, des polynucléotides et des petites molécules organiques hydrophobes, hydrophiles ou amphiphiles ;
- et/ou des nutriments ;
- et/ou de produits cosmétiques ou phytosanitaires ;
ce procédé étant caractérisé en ce qu'il consiste essentiellement à mettre en oeuvre au moins un homopolyaminoacide tel que défini ci-dessus et/ou la composition elle aussi décrite supra.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

Suivant une variante particulière de l'invention, cette méthode de traitement thérapeutique consiste essentiellement à mettre la composition telle que décrite supra sous forme de solution dans un solvant biocompatible puis à l'injecter en sous-cutané, intramusculaire ou dans une tumeur, de préférence de manière à ce qu'elle forme un dépôt sur le site d'injection.

L'invention sera mieux comprise et ses avantages et variantes de mise en oeuvre ressortiront bien des exemples qui suivent et qui décrivent la synthèse des homopolyaminoacides branchés, leur transformation en système de vectorisation de PA (suspension colloïdale aqueuse stable) et la démonstration de la capacité d'un tel système de s'associer à une protéine pour former des compositions pharmaceutiques.

### EXEMPLES :

### Exemple 1 : Synthèse du polymère (1) de type I

### Indices et groupements :

m=108,n=8,o=4,p=15
GH = D,L-alpha-Tocophérol (T)
A = -CH₂-CH₂- (glutamate) ; E = NH₂, E' = leucinamide ; C, D = liaisons directes

### Etape 1 : Polymérisation des branches et couplage sur le pGluT

*Solution 1:* Dans un ballon tricol de 50 ml sous flux d'azote, 3,4 g de NCA GluOBn sont dissous dans 9 ml de NMP à 25°C. 89 mg de leucinamide sont ajoutés au milieu réactionnel. La polymérisation est arrêtée à 80% de conversion du NCA par refroidissement du milieu réactionnel à 0°C.

*Solution 2 :* En parallèle, 3,5 g d'un poly(acide glutamique) de degré de polymérisation (DP) 120 et greffé à 7% molaire de façon statistique avec de l'alpha-tocophérol synthétique (obtenu selon le mode opératoire décrit dans WO-A-03/104303), sont solubilisés, par chauffage à 80°C, dans 44 ml de DMF dans un ballon tricol de 100 ml. A cette solution refroidie à -15°C, sont ajoutés 128 ml d'isobutyl chloroformate puis 109 ml de N-méthyl morpholine. Le milieu réactionnel est agité 15 minutes en laissant la température remonter à 0°C. Le milieu réactionnel est à nouveau refroidi à-15°C avant de procéder à l'ajout de la solution 1. La température remonte à l'ambiante puis le milieu est agité 2 heures à 40°C. Le polymère est précipité dans de l'eau acidifié (pH<2, 315 ml), filtré et lavé avec de l'eau acide (2*157 ml) et de l'éther iso (2*157 ml) puis séché à l'étuve sous vide à 40°C. 5,8 g du polymère branché intermédiaire sont obtenus, soit 100 % de rendement. Le degré de polymérisation des branches déterminé par RMN ¹H dans le TFA-d est de 17.

### Etapes 2 et 3 : Hydrolyse des esters benzyliques des branches et neutralisation

5,5 g du précédent polymère sont ensuite dissous, à température ambiante, dans 42 ml de TFA (c_{poly} = 130 mg/ml). Cette solution est refroidie à 0°C avant de procéder à l'ajout des 6,8 ml de HBr (30 % dans l'acide acétique). Le milieu est ensuite remis à température ambiante pour 3 heures. La fin de la réaction est contrôlée par RMN ¹H dans le TFA-d et le milieu réactionnel est versé dans 300 ml d'eau contenant de la glace. Le précipité est filtré sur fritté. Le polymère est resolubilisé dans le THF (42 ml) puis précipité dans de l'éther diisopropylique, filtré et lavé à l'éther diisopropylique (3* 85 ml). Le produit est enfin séché dans une étuve sous vide à 40°C. 3,9 g du polymère (1) sous sa forme polyacide sont obtenus (soit 82 % de rendement).
Le polymère, mis en suspension dans de l'eau déminéralisée, est neutralisé par ajout d'une solution de NaOH 1N. La neutralisation est terminée lorsque tout le polymère s'est solubilisé et que le pH se situe autour de 7,4.

Le pourcentage de tocophérol déterminé par RMN ¹H dans le TFA-d est de 4,6%. Le Mn (déterminé par GPC NMP) est de 59,8 kg/mol en équivalents PMMA.

### Exemple 2 : synthèse du polymère (2) de type (I)

### Indices et groupements :

m=105,n=8,o=7,p=15
GH = D,L-alpha-Tocophérol (T)
A = -CH₂-CH₂- (glutamate) ; E = NH₂, E' = diester éthylique de l'acide glutamique ; C, D = liaisons directes

### Etape 1: Polymérisation des branches et couplage sur le p Glu T

*Solution 1 :* Dans un ballon tricol de 50 ml sous flux d'azote, 5,9 g de NCA GluOBn sont dissous dans 14 ml de NMP à 25°C. 243 mg du diester éthylique de l'acide glutamique solubilisés dans 1 ml de NMP sont ajoutés au milieu réactionnel. La polymérisation est arrêtée à 80 % de conversion du NCA par refroidissement du milieu réactionnel à 0°C.

***Solution 2: En parallèle, 3,0 g d'un poly(acide glutamique) de dégré de polymérisation*** 120 et greffé à 7 % molaire de façon statistique avec de l'alpha-tocophérol synthétique (obtenu selon le mode opératoire décrit dans WO-A-03/104303), sont solubilisés, par chauffage à 80°C, dans 44 ml de DMF dans un ballon tricol de 100 ml. A cette solution refroidie à -15°C, sont ajoutés 219 ml d'isobutyl chloroformate puis 186 ml de N-méthyl morpholine. Le milieu réactionnel est agité 15 minutes en laissant la température remonter à 0°C. Le milieu réactionnel est à nouveau refroidi à -15°C avant de procéder à l'ajout de la solution 1. La température remonte à l'ambiante puis le milieu est agité 2 heures à 40°C. Le polymère est précipité dans de l'eau acidifié (pH<2, 315 ml), filtré sur fritté et lavé avec de l'eau acide (2*157 ml) et de l'éther iso (2*157 ml) puis séché à l'étuve sous vide à 40°C. 6,5 g du polymère intermédiaire. Le degré de polymérisation des branches déterminé par RMN ¹H dans le TFA-d est de 19.

### Etapes 2 et 3 : Hydrolyse des esters benzyliques des branches et neutralisation

6,2 g du précédent polymère sont ensuite dissous, à température ambiante, dans 48 ml de TFA (c_{poly} = 130 mg/ml). Cette solution est refroidie à 0°C avant de procéder à l'ajout des 11,5 ml de HBr (30 % dans l'acide acétique). Le milieu est ensuite remis à température ambiante pour 3 heures. La fin de la réaction est contrôlée par RMN ¹H dans le TFA-d et le milieu réactionnel est versé dans 335 ml d'eau contenant de la glace. Le précipité est filtré sur fritté P4. Le polymère est resolubilisé dans du THF (48 ml) puis précipité dans de l'éther diisopropylique (480 ml), filtré sur fritté et lavé à l'éther diisopropylique (2 * 48 ml). Le produit est enfin séché dans une étuve sous vide à 40°C. 3,7 g du polymère (2) sont obtenus (soit 76 % de rendement).

Le polymère, mis en suspension dans de l'eau déminéralisée, est neutralisé par ajout d'une solution de NaOH 1N (de façon à ce que le pH ne dépasse jamais la valeur de 8). La neutralisation est terminée lorsque tout le polymère s'est solubilisé et que le pH se situe autour de 7,4.

Le pourcentage de tocophérol déterminé par RMN ¹H dans le TFA-*d* est de 3,5 %. Le Mn (déterminé par GPC NMP) est de 42,4 kg/mol en équivalents PMMA.

### Exemple 3 : synthèse du polymère (3) de type (I)

### Indices et groupements :

m=105,n=8,o=7,p=8
GH = D,L-alpha-Tocophérol (T)
A = -CH₂-CH₂- (glutamate) ; E = NH₂, E' = leucinamide ; C, D = liaisons directes

4,1 g de ce polymère ont été synthétisés selon le procédé décrit dans l'exemple 1. Le degré de polymérisation des branches déterminé par RMN ¹H dans le TFA-d est de 10. Le pourcentage de tocophérol déterminé par RMN ¹H dans le TFA-*d* est de 4,3 %. Le Mn (déterminé par GPC NMP) est de 66,3 kg/mol en équivalents PMMA.

### Exemple 4 : synthèse du polymère (4) de type (I)

### Indices et groupements :

m=25,n=7,o=3,p=15
GH = D,L-alpha-Tocophérol (T)
A = -CH₂-CH₂- (glutamate) ; E = NH₂, E' = leucinamide ; C, D = liaisons directes

3,9 g de ce polymère ont été synthétisés selon le procédé décrit dans l'exemple 2. Le degré de polymérisation des branches déterminé par RMN¹H dans le TFA-*d* est de 18. Le pourcentage de tocophérol déterminé par RMN ¹H dans le TFA-d est de 7,0 %. Le Mn (déterminé par GPC NMP) est de 24,1 kg/mol en équivalents PMMA.

### Exemple 5 : synthèse du polymère (5) de type (II)

### Indices et groupements :

m=103,n=5,o=12,p=30,q=1
GH = D,L-alpha-Tocophérol (T)
A = -CH₂-CH₂- (glutamate) ; E = NH₂, E' = leucinamide ; C, D = liaisons directes

### Etape 1 : Polymérisation des branches, couplage sur le pGluOH et hydrolyse des esters des branches

*Solution 1:* Dans un ballon tricol de 250 ml sous flux d'azote, 20,0 g de NCA GluOMe sont dissous dans 86 ml de NMP à 40° C. 345 mg de leucinamide dissous dans 4 ml de NMP sont ajoutés au milieu réactionnel. La polymérisation est arrêtée à 80 % de conversion du NCA par refroidissement du milieu réactionnel à 0°C.

*Solution 2 :* En parallèle, 3,3 g d'un poly(acide glutamique) de DP 120, sont solubilisés, par chauffage à 80°C, dans 52 ml de DMF dans un ballon tricol de 500 ml. A cette solution refroidie à 0° C, sont ajoutés 502 ml d'isobutyl chloroformate puis 426 ml de N-méthyl morpholine. Le milieu réactionnel est agité à 0° C pendant la polymérisation de la solution 1. Avant de procéder à l'ajout de la solution 1, 71 ml de N-méthyl morpholine sont ajoutés. La température remonte à l'ambiante et le milieu est agité 4,5 heures à cette température. La température du milieu est ensuite portée à 80°C. A cette température, une solution de NMP/HCl 35 % est ajoutée au goutte à goutte (14,5 ml /28,5 ml) et la pression dans le ballon est abaissée à 600 mbars. Après 5 jours d'hydrolyse, le polymère est précipité dans de l'eau acidifié (pH<2,710 ml), centrifugé et lavé avec de l'eau acide (200 ml) puis de l'eau (2*200 ml). Le polymère est filtré sur fritté puis séché à l'étuve sous vide à 40°C. 12,3 g du polymère intermédiaire sont obtenus (soit 90 % de rendement). Le degré de polymérisation des branches déterminé par RMN ¹H dans le TFA-d est de 50.

### Etapes 2 et 3 : Greffage du tocophérol et la neutralisation

5,0 g du précédent polymère sont dissous, à 80°C, dans 100 ml de DMF (c_{poly} = 50 mg/ml). A cette solution sont ajoutés 47 mg de DMAP solubilisés dans 0,6 ml de DMF. Le milieu est maintenu à 80°C pendant 18 heures. La température est ensuite abaissée à 15°C et sont ajoutés dans l'ordre, une solution de tocophérol (834 mg dans 2,6 ml de DMF), une solution de DMAP (50 mg dans 0,6 ml de DMF) et la DIPC (546 mg). Le milieu réactionnel est agité à cette température pendant 3,5 heures. Le milieu réactionnel est bloqué avec de l'HCl 35 % (4,0 ml) et le polymère ensuite précipité dans un milieu biphasique eau acide (400 ml, 60 g de NaCl et pH < 2) éther diisopropylique (80 ml). Le produit est lavé trois fois avec un mélange biphasique eau acide / éther diisopropylique (3 * 300 / 80 ml) puis deux fois avec de l'éther diisopropylique (2 * 300 ml). Le produit est enfin séché dans une étuve sous vide à 40°C. 5,1 g du polymère (5) sont obtenus (soit 87 % de rendement).

Le polymère, mis en suspension dans de l'eau déminéralisée, est neutralisé par ajout d'une solution de NaOH 1N.La neutralisation est terminée lorsque tout le polymère s'est solubilisé et que le pH se situe autour de 7,4.
Le pourcentage de tocophérol déterminé par RMN ¹H dans le TFA-*d* est de 4,5 %. Le Mn (déterminé par GPC NMP à 70°C) est de 248,6 kg/mol en équivalents PMMA.

### Exemple 6 : Synthèses des composés C1, C2, C3 et C4 de type linéaire

Ces composés sont obtenus par la méthode décrite dans la demande WO-A-03/104303. Les caractéristiques de ces polymères sont données dans le tableau ci-dessous.

### Exemple 7 : Etudes des propriétés de solubilité et de viscosité

A titre de comparaison des propriétés et pour démontrer l'invention, on réalise une mesure de la viscosité du polymère à pH 7,4 et à une osmolalité de 300 mOsmol en fonction de la concentration. On mesure ensuite la concentration limite d'agrégation Cη (g/l): concentration à partir de laquelle la viscosité augmente très rapidement. Les résultats et les caractéristiques des polymères de l'invention et les composés comparatifs de l'art antérieur sont rassemblés dans le tableau 1 ci-dessous.

**TABLEAU 1**

| **Exemple** | **Type** | **DP total** | **% GH molaire** | **Cη (g/l)** |
|---|---|---|---|---|
| 1 | Branché type I | 180 | 4,6 | 35 |
| 2 | Branché type I | 230 | 3,5 | 70 |
| 3 | Branché type I | 180 | 4,3 | 40 |
| 4 | Branché type I | 85 | 7,0 | 100 |
| 5 | Branché type II | 480 | 4,5 | 20 |
| C1 | Linéaire | 120 | 7,3 | 20 |
| C2 | Linéaire | 120 | 4,0 | 28 |
| C3 | Linéaire | 220 | 5,0 | 25 |
| C4 | Linéaire | 500 | 4,1 | 10 |

| | | | | |
|---|---|---|---|---|
| Cη est la concentration limite d'agrégation : concentration à partir de laquelle la viscosité augmente très rapidement. Le DP total correspond au dégré de polymérisation du polymère soit branché, soit linéaire. | | | | |

La comparaison des viscosités, illustrées par les valeurs Cη, montre qu'il est bien plus facile d'obtenir une solution concentrée avec les polymères de l'invention. Notamment, la comparaison des polymères ayant un squelette de taille analogue et un taux de greffage équivalent, (1), (2) et (3) versus C1 et C2 d'une part et, (5) et C4 d'autre part, démontre clairement cette différence. Cette propriété permet donc de réaliser des formulations ayant des concentrations élevées en polymères, pouvant augmenter ainsi le rapport polymère/principe actif, tout en assurant une bonne injectabilité.

### Exemple 8 : Etude d'association avec l'insuline

On prépare une solution aqueuse contenant 10 mg de polymère par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 KDa, 15 minutes sous 10000G à 18 °C). L'insuline libre récupérée dans le filtrat est ensuite dosée par CLHP (Chromatographie Liquide Haute Performance) et l'on déduit la quantité d'insuline associée. Les résultats sont donnés dans le tableau 2 ci-dessous.

**TABLEAU 2**

| Polymère | % association |
|---|---|
| 1 | 96 |
| 2 | 90 |
| 5 | 99 |

Les résultats démontrent que les polymères de l'invention sont capables d'associer fortement l'insuline pour donner des suspensions colloïdales de taille supérieure à 100 Kda et les taux d'association avec l'insuline sont très élevés. La capacité d'association de ces polymères les rend aptes à être utilisés comme agents de vectorisation.

## Revendications

1. Polyaminoacide comprenant des unités acide aspartique et/ou des unités acide glutamique, dont certaines sont porteuses d'un ou de plusieurs groupements hydrophobes (GH), identiques ou différents entre eux,
**caractérisé en ce qu'**il comprend au moins un type de chaîne principale Cp polyaminoacide comportant des unités acide aspartique et/ou des unités acide glutamique et présentant une ou plusieurs branches B polyaminoacide(s) identiques ou différentes entre elles, comportant des unités acide aspartique et/ou des unités acide glutamique.

2. Polyaminoacide selon la revendication 1, **caractérisé en ce que** les groupements hydrophobes sont liés :
→ (I) à la chaîne principale Cp seulement,
→ (II) à la chaîne principale Cp et à la/aux branches B,
→ (III) ou à la/aux branches B seulement.

3. Polyaminoacide selon l'une des revendications précédentes, **caractérisé en ce que** les groupements hydrophobes GH comportent 8 à 30 atomes de carbone.

4. Polyaminoacide selon la revendication 3, **caractérisé en ce que** les groupements hydrophobes GH sont choisis dans le groupe comprenant :
• les alkyles linéaires ou ramifiés en C8 à C30 pouvant comporter éventuellement au moins une insaturation et/ou au moins un hétéroatome,
• les alkylaryles ou arylalkyles en C8 à C30 pouvant comporter éventuellement au moins une insaturation et/ou au moins un hétéroatome,
• et les (poly)cycliques en C8 à C30 pouvant comporter éventuellement au moins une insaturation et/ou au moins un hétéroatome.

5. Polyaminoacide selon l'une des revendications 1 à 4, **caractérisé en ce que** les groupements hydrophobes GH sont dérivés d'un groupement choisi dans le groupe comprenant : l'octanol, le dodécanol, le tétradécanol, l'héxadécanol, l'octadécanol, l'oleylalcool, le tocophérol, le cholestérol ou l'acide lithocholique.

6. Polyaminoacide selon l'une des revendications précédentes, **caractérisé en ce qu'**il comprend des unités d'alpha-L-glutamate et/ou d'acide alpha-L-glutamique ou des unités d'alpha-L-aspartate et/ou d'acide alpha-L-aspartique.

7. Polyaminoacide selon l'une des revendications précédentes, **caractérisé en ce qu'**il répond à l'une des formules générales (**I**), (**II**) et (**III**) suivantes : 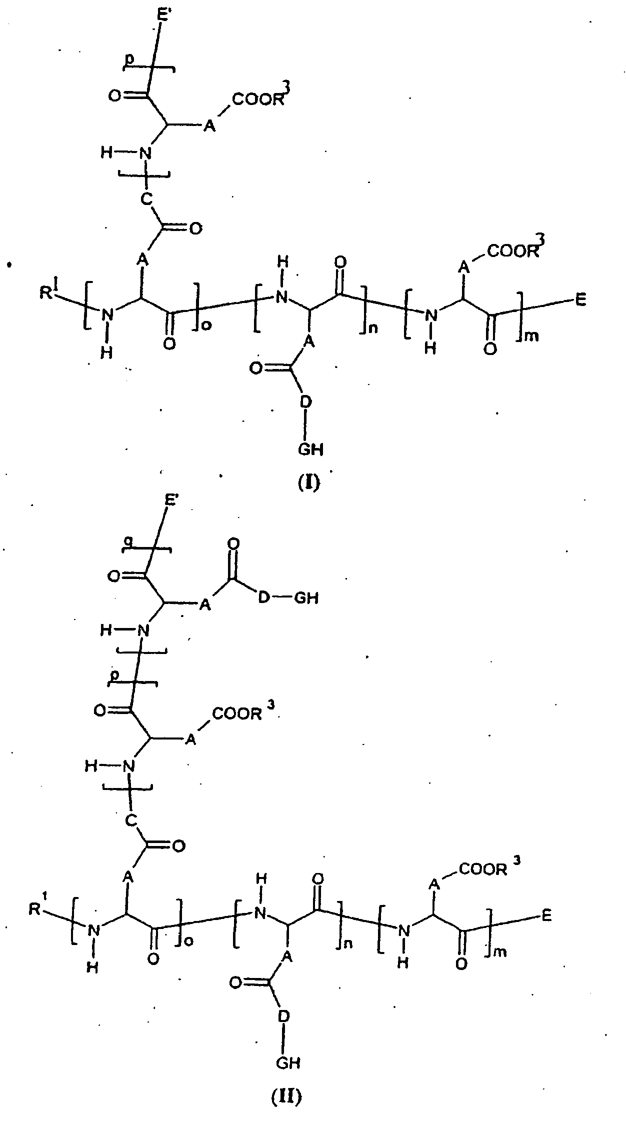 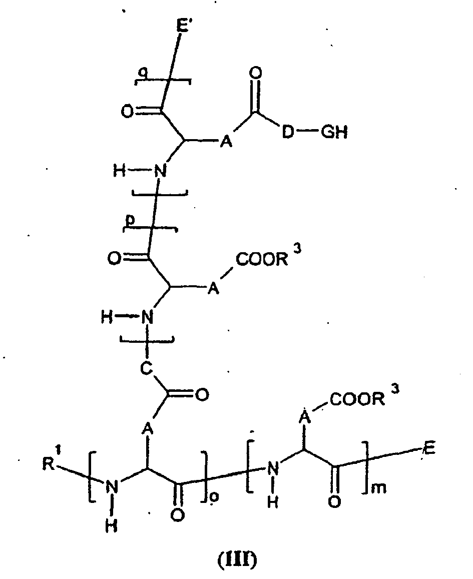 dans desquelles :
■ A représente indépendamment un -CH₂- ou -CH₂-CH₂-;
■ R¹ représente un H, un groupe acyle linéaire en C2 à C10 ou ramifié en C3 à C10, ou un pyroglutamate;
■ E, E' représentent indépendamment :
- un OR³, R³ répondant à la définition donnée ci-dessous,
- un groupement NHR² dans laquelle R² représente un H, un alkyle linéaire en C2 à C10 ou ramifié en C3 à C10 ou un benzyle,
- une unité acide aminé terminale liée par l'azote ;
■ R³ est un H ou une entité cationique, sélectionnée dans le groupe comprenant :
- les cations métalliques choisis dans le sous-groupe comprenant : le sodium, le potassium, le calcium, le magnésium ;
- les cations organiques choisis dans le sous-groupe comprenant :
• les cations à base d'amine,
• les cations à base d'oligoamine,
• les cations à base de polyamine,
• les cations à base d'acide(s) aminé(s) choisis dans la classe comprenant les cations à base de lysine ou d'arginine,
- ou les polyaminoacides cationiques choisis dans le sous-groupe comprenant la polylysine ou l'oligolysine;
■ C représente une liaison directe ou un groupement de liaison choisi parmi un reste d'acide aminé ou un hydroxyalcool comportant de 1 à 6 atomes de carbones;
■ les groupements D-GH représentent chacun indépendamment les uns des autres un radical dans lequel :
- D est un groupement de liaison -O-, -NH-, -N-alkyle- (C1 à C5), un résidu d'acide aminé, un diol, une diamine, un aminoalcool ou un hydroxyacide comportant de 1 à 6 atomes de carbone, et
- GH représente un groupement hydrophobe comportant 8 à 30 atomes de carbone;
• alkyles linéaires ou ramifiés en C8 à C30 pouvant comporter au moins une insaturation et/ou au moins un hétéroatome, ou
• alkylaryles ou arylalkyle en C8 à C30 pouvant comporter au moins une insaturation et/ou au moins un hétéroatome, ou
• (poly)cycliques en C8 à C30 pouvant comporter au moins une insaturation et/ou au moins un hétéroatome.
■(n+q)/(q+p+o+n+m) est défini comme le taux de greffage molaire des groupements hydrophobes GH et varie de 0,5 à 90 % molaire ;
■ q+p+o+n+m varie de 20 à 5000 ;
■ o+n+m varie de 10 à 500 ;
■ o/(o+n+m) est défini comme le taux de greffage des branches et varie entre 1 et 50 % molaire ;
■ la chaîne principale Cp de ces polyaminoacides (I) (II) (III) est constituée par les motifs récurrents -[ ]ₒ-[ ]ₙ-[ ]ₘ- ou -[ ]ₒ-[ ]ₘ- et leurs branches B par les motifs récurrents -[ ]ₚ- ou-[ ]ₚ-[ ]_{q}-;
■ et les groupements hydrophobes GH et les branches B sont disposés de façon aléatoire.

8. Polyaminoacide selon l'une quelconque des revendications précédentes, **caractérisé en ce que** sa masse molaire se situe entre 2 000 et 800 000 g/mole.

9. Composition pharmaceutique, cosmétique, diététique ou phytosanitaire comprenant au moins un polyaminoacide selon l'une quelconque des revendications 1 à 8.

10. Composition selon la revendication 9, **caractérisée en ce qu'**elle comprend au moins un principe actif.

11. Composition; selon la revendication 10, **caractérisé en ce que** le principe actif est associé au(x) polyaminoacide(s) par une ou plusieurs liaisons autre(s) qu'une (ou que des) liaison(s) chimique(s) covalente(s).

12. Composition selon la revendication 10 ou 11, **caractérisée en ce que** le principe actif est une protéine, une glycoprotéine, une protéine liée à une ou plusieurs chaînes polyalkylèneglycol, un polysaccharide, un liposaccharide, un oligonucléotide, un polynucléotide ou un peptide.

13. Composition selon l'une quelconque des revendications 9 à 12, **caractérisée en ce qu'**elle peut être administrée par voie orale, parentérale, nasale, vaginale, oculaire, sous-cutanée, intraveineuse, intramusculaire, intradermique, intrapéritonéale, intracérébrale ou buccale.

14. Composition selon l'une quelconque des revendications 9 à 13, **caractérisée en ce qu'**elle est sous forme d'un gel, d'une solution, d'une émulsion, de micelles, de nanoparticules, de microparticules, d'une poudre ou d'un film.

15. Composition selon l'une quelconque des revendications 9 à 14, **caractérisée en ce qu'**elle est une suspension colloïdale de nanoparticules et/ou de microparticules et/ou de micelles de polyaminoacides, dans une phase aqueuse.

16. Composition selon l'une quelconque des revendications 9 à 15, **caractérisée en ce qu'**elle est sous forme de solution dans un solvant biocompatible et **en ce qu'**elle peut être injectée par voie sous-cutanée, intramusculaire ou dans une tumeur.

17. Composition selon la revendication 16, **caractérisée en ce qu'**elle est apte à former un dépôt sur le site d'injection.

18. Procédé de préparation :
• de médicaments, pour administration orale, nasale, vaginale, oculaire, sous-cutanée, intraveineuse, intramusculaire, intradermique, intrapéritonéale ou intracérébrale, les principes actifs de ces médicaments pouvant être des protéines, des glycoprotéines, des protéines liées à une ou plusieurs chaînes polyalkylèneglycol, des peptides, des polysaccharides, des liposaccharides, des oligonucléotides, des polynucléoides et des molécules organiques non protéiniques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un polyaminoacide selon l'une quelconque des revendications 1 à 8 et/ou la composition selon l'une quelconque des revendications 9 à 17.

## Claims

1. Polyamino acid comprising aspartic acid units and/or glutamic acid units, certain of which bear one or more hydrophobic groups (GH), which are identical to or different from each other,
**characterized in that** it comprises at least one type of main polyamino acid chain Cp comprising aspartic acid units and/or glutamic acid units and having one or more polyamino acid branches B which are identical to or different from each other, comprising aspartic acid units and/or glutamic acid units.

2. Polyamino acid according to claim 1, **characterized in that** the hydrophobic groups are bound:
→ (I) only to the main chain Cp,
→ (II) to the main chain Cp and to the branch(es) B,
→ (III) or only to the branch(es) B.

3. Polyamino acid according to one of the previous claims, **characterized in that** the hydrophobic groups -GH-comprise 8 to 30 carbon atoms.

4. Polyamino acid according to claim 3, **characterized in that** the hydrophobic groups GH are chosen from the group comprising:
• the linear or branched C8 to C30 alkyls which may optionally comprise at least one unsaturation and/or at least one heteroatom,
• the C8 to C30 alkylaryls or arylalkyls which may optionally comprise at least one unsaturation and/or at least one heteroatom,
• and the C8 to C30 (poly)cyclics which may optionally comprise at least one unsaturation and/or at least one heteroatom.

5. Polyamino acid according to one of claims 1 to 4, **characterized in that** the hydrophobic groups GH are derived from a group chosen from the group comprising: octanol, dodecanol, tetradecanol, hexadecanol, octadecanol, oleyl alcohol, tocopherol, cholesterol or lithocholic acid.

6. Polyamino acid according to one of the previous claims, **characterized in that** it comprises alpha-L-glutamate and/or alpha-L-glutamic acid units or alpha-L-aspartate and/or alpha-L-aspartic acid units.

7. Polyamino acid according to one of the previous claims **characterized in that** it corresponds to one of the following general formulae (I), (II) and (III): 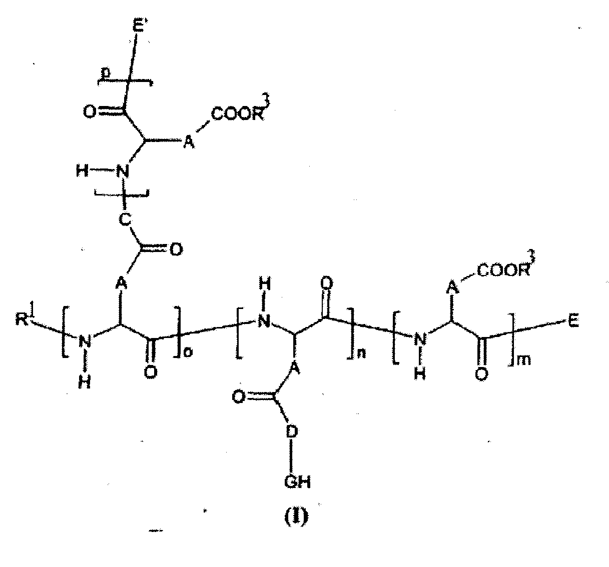 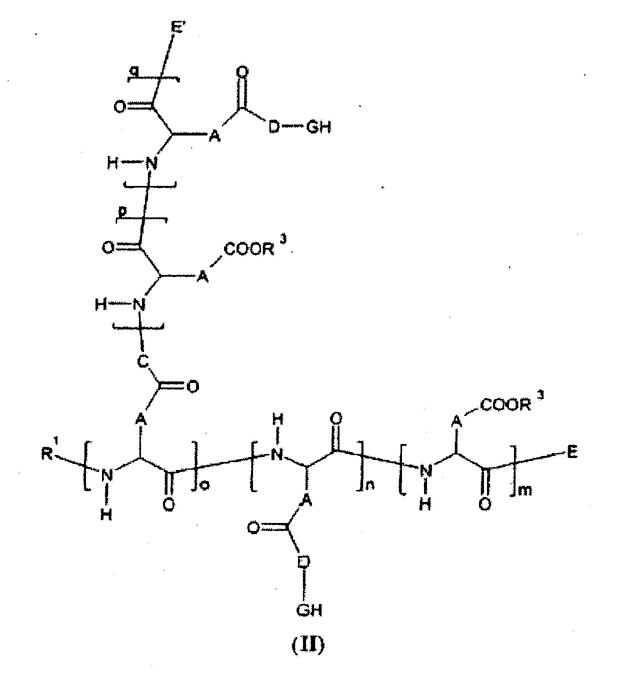 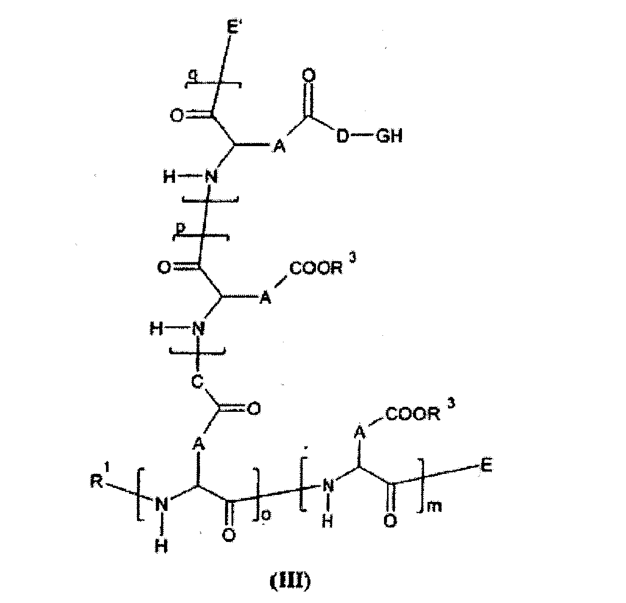 in which:
■ A represents independently a -CH₂- or -CH₂-CH₂-;
■ R¹ represents an H, a linear C2 to C10 or branched C3 to C10 acyl group, or a pyroglutamate;
■ E, E' represent independently:
- an OR³, R³ corresponding to the definition given below,
- an NHR² group in which R² represents an H, a linear C2 to C10 or branched C3 to C10 alkyl or a benzyl,
- a terminal amino acid unit bound by the nitrogen;
■ R³ is an H or a cationic entity, selected from the group comprising:
- metallic cations chosen from the subgroup comprising: sodium, potassium, calcium, magnesium;
- organic cations chosen from the subgroup comprising:
• cations based on amine,
• cations based on oligoamine,
• cations based on polyamine,
• cations based on amino acid(s) chosen from the class comprising cations based on lysine or arginine,
- or cationic polyamino acids chosen from the subgroup comprising polylysine or oligolysine;
■ C represents a direct bond or a binding group chosen from an amino acid residue or a hydroxyalcohol comprising 1 to 6 carbon atoms;
■ the D-GH groups each represent independently of each other a radical in which:
- D is a binding unit -O-, -NH-, -N-alkyl- (C1 to C5), an amino acid residue, a diol, a diamine, an amino alcohol or a hydroxy acid comprising 1 to 6 carbon atoms, and
- GH represents a hydrophobic group comprising 8 to 30 carbon atoms;
• linear or branched C8 to C30 alkyls which may comprise at least one unsaturation and/or at least one heteroatom, or
• C8 to C30 alkylaryls or arylalkyls which may comprise at least one unsaturation and/or at least one heteroatom, or
• C8 to C30 (poly)cyclics which may comprise at least one unsaturation and/or at least one heteroatom.
■ (n+q)/(q+p+o+n+m) is defined as the molar grafting rate of the hydrophobic groups GH and varies from 0.5 to 90 molar %;
■ q+p+o+n+m varies from 20 to 5000;
■ o+n+m varies from 10 to 500;
■ o/(o+n+m) is defined as the molar grafting rate of the branches and varies between 1 and 50 molar %;
■ the main chain Cp of these polyamino acids (I) (II) (III) is constituted by the recurrent units - [ ]ₒ- [ ]ₙ-[ ]ₘ- or - [ ]ₒ- [ ]ₘ- and their branches B by the recurrent units - [ ]ₚ- or - [ ]ₚ- [ ]_{q}-;
■ and the hydrophobic groups GH and the branches B are arranged in a random manner.

8. Polyamino acid according to any one of the previous claims, **characterized in that** its molar mass is situated between 2,000 and 800,000 g/mole.

9. Pharmaceutical, cosmetic, dietetic or phytosanitary composition comprising at least one polyamino acid according to any one of claims 1 to 8.

10. Composition according to claim 9, **characterized in that** it comprises at least one active ingredient.

11. Composition according to claim 10, **characterized in that** the active ingredient is combined with the polyamino acid(s) by one or more bonds other than one (or more) covalent chemical bond(s).

12. Composition according to claim 10 or 11, **characterized in that** the active ingredient is a protein, a glycoprotein, a protein bound to one or more polyalkylene glycol chains, a polysaccharide, a liposaccharide, an oligonucleotide, a polynucleotide or a peptide.

13. Composition according to any one of claims 9 to 12, **characterized in that** it can be administered by oral, parenteral, nasal, vaginal, ocular, sub-cutaneous, intravenous, intramuscular, intradermal, intraperitoneal, intracerebral or buccal route.

14. Composition according to any one of claims 9 to 13, **characterized in that** it is in the form of a gel, a solution, an emulsion, micelles, nanoparticles, microparticles, a powder or a film.

15. Composition according to any one of claims 9 to 14, **characterized in that** it is a colloidal suspension of nanoparticles and/or of microparticles and/or of micelles of polyamino acids, in an aqueous phase.

16. Composition according to any one of claims 9 to 15, **characterized in that** it is in the form of a solution in a biocompatible solvent and **in that** it can be injected by sub-cutaneous or intramuscular route or into a tumour.

17. Composition according to claim 16, **characterized in that** it is capable of forming a deposit on the injection site.

18. Method for the preparation:
• of medicaments, for oral, nasal, vaginal, ocular, sub-cutaneous, intravenous, intramuscular, intradermal, intraperitoneal or intracerebral administration, the active ingredients of these medicaments being proteins, glycoproteins, proteins bound to one or more polyalkylene glycol chains, peptides, polysaccharides, liposaccharides, oligonucleotides, polynucleotides and hydrophobic, hydrophilic or amphiphilic non-protein organic molecules;
• and/or of nutrients;
• and/or of cosmetic or phytosanitary products; **characterized in that** it essentially consists of utilizing at least one polyamino acid according to any one of claims 1 to 8 and/or the composition according to any one of claims 9 to 17.

## Patentansprüche

1. Polyaminosäure, die Asparaginsäureeinheiten und/oder Glutaminsäureeinheiten umfaßt, von denen manche eine oder mehrere hydrophobe Gruppen (GH), die gleich oder verschieden voneinander sind, tragen,
**dadurch gekennzeichnet, daß** sie mindestens eine Art von Polyaminosäure-Hauptkette Cp umfaßt, die Asparaginsäureeinheiten und/oder Glutaminsäureeinheiten umfaßt und eine oder mehrere Polyaminosäure-Verzweigungen B, die gleich oder voneinander verschieden sind, und die Asparaginsäureeinheiten und/oder Glutaminsäureeinheiten umfassen, aufweist.

2. Polyaminosäure nach Anspruch 1, **dadurch gekennzeichnet, daß** die hydrophoben Gruppen
→ (I) an nur an die Hauptkette Cp,
→ (II) an die Hauptkette Cp und an die Verzweigung(en) B,
→ (III) oder nur an die Verzweigung(en) B gebunden sind.

3. Polyaminosäure nach einem der vorhergehenden Ansprüche, **dadurch gekennzeichnet, daß** die hydrophobe GH-Gruppen 8 bis 30 Kohlenstoffatome umfaßen.

4. Polyaminosäure nach Anspruch 3, **dadurch gekennzeichnet, daß** die hydrophoben GH-Gruppen aus der Gruppe, umfassend:
• lineare oder verzweigte C8- bis C30-Alkyle, die gegebenenfalls mindestens eine Unsättigung und/oder mindestens ein Heteroatom umfassen können,
• C8- bis C30-Alkylaryle oder -Arylalkyle, die gegebenenfalls mindestens eine Unsättigung und/oder mindestens ein Heteroatom umfassen können,
• sowie C8- bis C30-(Poly)zyklen, die gegebenenfalls mindestens eine Unsättigung und/oder mindestens ein Heteroatom umfassen können,
ausgewählt sind.

5. Polyaminosäure nach einem der Ansprüche 1 bis 4, **dadurch gekennzeichnet, daß** sich die hydrophobe GH-Gruppen von einem aus der Gruppe, umfassend: Octanol, Dodecanol, Tetradecanol, Hexadecanol, Octadecanol, Oleylalkohol, Tocopherol, Cholesterol oder Lithocholinsäure, ausgewählten Gruppen abstammen.

6. Polyaminosäure nach einem der vorhergehenden Ansprüche, **dadurch gekennzeichnet, daß** sie alpha-L-Glutamat- und/oder alpha-L-Glutaminsäure- oder alpha-L-Aspartat- und/oder alpha-L-Asparaginsäureeinheiten umfaßt.

7. Polyaminosäure nach einem der vorhergehenden Ansprüche, **dadurch gekennzeichnet, daß** sie einer der folgenden allgemeinen Formeln (**I**), (**II**), (**III**) entspricht: 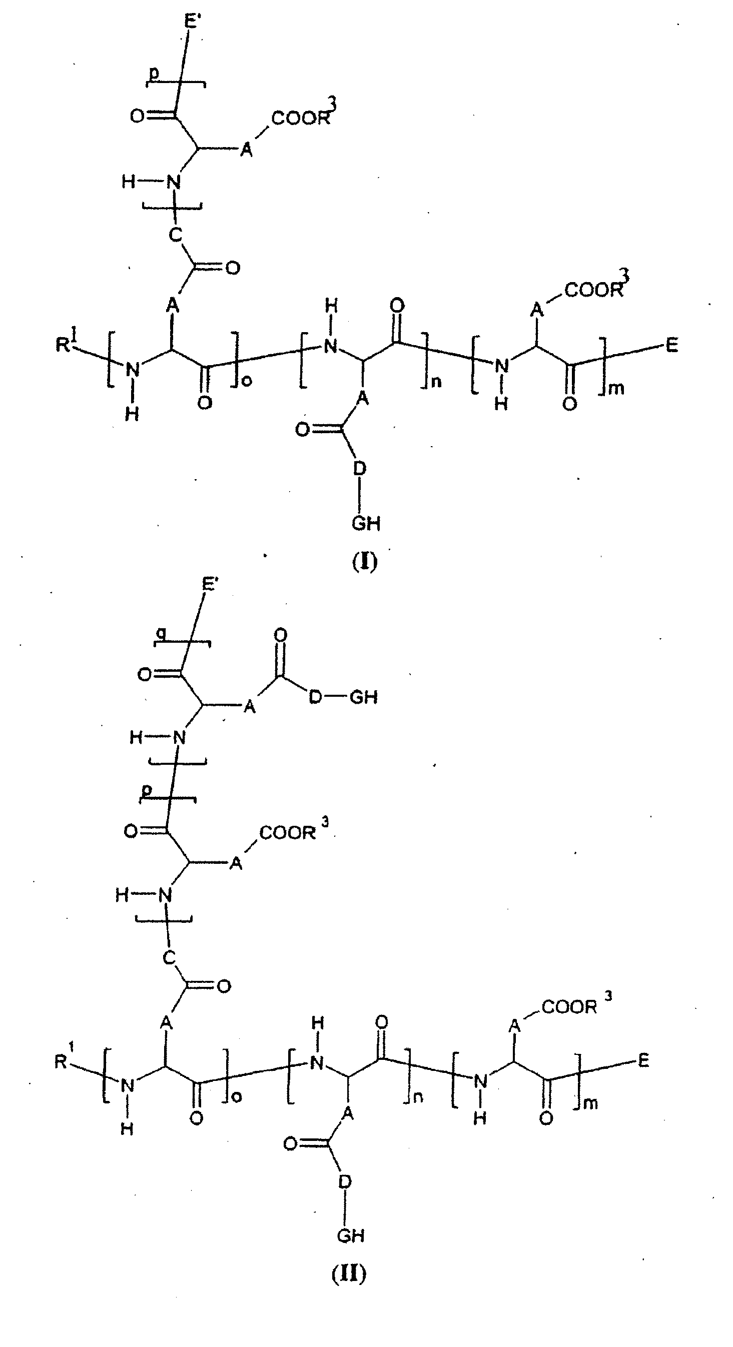 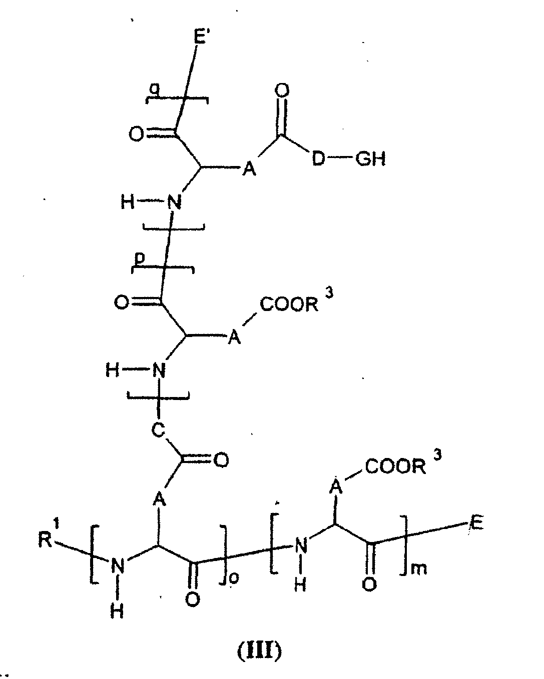 wobei:
■ A unabhängig für ein -CH₂- oder -CH₂-CH₂-steht;
■ R¹ für ein H, eine lineare C2- bis C10-Acylgruppe oder eine verzweigte C3- bis C10-Acylgruppe oder ein Pyroglutamat steht;
■ E, E' unabhängig für :
- ein OR³, wobei R³ wie unten definiert ist,
- eine NHR²-Gruppe, in der R² für ein H, ein lineares C2- bis C10-Alkyl oder ein verzweigtes C3- bis C10-Alkyl oder ein Benzyl steht,
- eine über den Stickstoff gebundene terminale Aminosäureeinheit stehen;
■ R³ ein H oder eine kationische Einheit ist, die ausgewählt ist aus der Gruppe, umfassend:
- metallische Kationen, ausgewählt aus der Untergruppe, umfassend: Natrium, Kalium, Calcium, Magnesium;
- organische Kationen, ausgewählt aus der Untergruppe, umfassend:
• Kationen auf der Basis von Amin,
• Kationen auf der Basis von Oligoamin,
• Kationen auf der Basis von Polyamin,
• Kationen auf der Basis von Aminosäure(en), ausgewählt aus der Klasse, umfassend Kationen auf der Basis von Lysin oder Arginin,
- oder kationische Polyaminosäuren, ausgewählt aus der Untergruppe, umfassend Polylysin oder Oligolysin;
■ C für eine direkte Bindung oder eine Bindungsgruppe, ausgewählt aus der Reihe Aminosäurerest oder Hydroxyalkohol, umfassend 1 bis 6 Kohlenstoffatome, steht;
■ die Gruppe D-GH jeweils unabhängig voneinander einen Rest bedeuten, in dem:
- D eine Bindungsgruppe -O-, -NH-, C1- bis C5-N-Alkyl, ein Aminosäurerest, ein Diol, ein Diamin, ein Aminoalkohol oder eine Hydroxysäure, umfassend 1 bis 6 Kohlenstoffatome, ist, und
- GH für eine hydrophobe Gruppe, umfassend 8 bis 30 Kohlenstoffatome, steht;
• lineare oder verzweigte C8- bis C30-Alkyle, die mindestens eine Unsättigung und/oder mindestens ein Heteroatom umfassend können, oder
• C8- bis C30-Alkylaryle oder -Arylalkyle mindestens eine Unsättigung und/oder, die mindestens ein Heteroatom umfassen können, oder
• C8- bis C30-(Poly)zyklen, die mindestens eine Unsättigung und/oder mindestens ein Heteroatom umfassen können,
■ (n+q)/(q+p+o+n+m) als molare Propfrate der hydrophoben GH-Gruppen definiert ist und von 0,5 bis 90 Mol-% variiert;
■ q+p+o+n+m von 20 bis 5000 variiert;
■ o+n+m von 10 bis 500 variiert;
■ o/(o+n+m) als molare Propfrate der Verzweigungen definiert ist und zwischen 1 und 50 Mol-% variiert;
■ die Hauptkette Cp dieser Polyaminosäuren (I) (II) (III) aus den Wiederholungseinheiten -[ ]ₒ-[ ]ₙ- [ ]ₘ- oder -[ ]ₒ- [ ]ₘ- und ihre Verzweigungen B aus den Wiederholungseinheiten - [ ]ₚ- oder -[ ]ₚ-[ ]q- bestehen;
■ und die hydrophoben GH-Gruppen und die Verzweigungen B zufallsmäßig angeordnet sind.

8. Polyaminosäure nach einem der vorhergehenden Ansprüche, **dadurch gekennzeichnet, daß** ihre Molmasse zwischen 2.000 und 800.000 g/mol liegt.

9. Pharmazeutische Zusammensetzung, kosmetische Zusammensetzung, diätetische Zusammensetzung oder Pflanzenschutzzusammensetzung, die mindestens eine Polyaminosäure nach einem der Ansprüche 1 bis 8 umfaßt.

10. Zusammensetzung nach Anspruch 9, **dadurch gekennzeichnet, daß** sie mindestens einen Wirkstoff umfaßt.

11. Zusammensetzung nach Anspruch 10, **dadurch gekennzeichnet, daß** der Wirkstoff mit der Polyaminosäure bzw. den Polyaminosäuren durch eine oder mehrere Bindungen verbunden ist, bei der bzw. denen es sich nicht um eine kovalente chemische Bindung bzw. nicht um kovalente chemische Bindungen handelt.

12. Zusammensetzung nach Anspruch 10 oder 11, **dadurch gekennzeichnet, daß** es sich bei dem Wirkstoff um ein Protein, ein Glykoprotein, ein an eine oder mehrere Polyalkylenglykolketten gebundenes Protein, ein Polysaccharid, ein Liposaccharid, ein Oligonukleotid, ein Polynukleotid oder eine Peptid handelt.

13. Zusammensetzung nach einem der Ansprüche 9 bis 12, **dadurch gekennzeichnet, daß** sie auf oralem, parenteralem, nasalem, vaginalem, okularem, subkutanem, intravenösem, intramuskulärem, intradermalem, intraperitonealem, intrazerebralem oder bukkalem Weg verabreicht werden kann.

14. Zusammensetzung nach einem der Ansprüche 9 bis 13, **dadurch gekennzeichnet, daß** sie in Form eines Gels, einer Lösung, einer Emulsion, von Mizellen, von Nanopartikeln, von Mikropartikeln, eines Pulvers oder eines Films vorliegt.

15. Zusammensetzung nach einem der Ansprüche 9 bis 14, **dadurch gekennzeichnet, daß** es sich um eine kolloidale Suspension von Nanopartikeln und/oder Mikropartikeln und/oder von Mizellen von Polyaminosäuren in einer wäßrigen Phase handelt.

16. Zusammensetzung nach einem der Ansprüche 9 bis 15, **dadurch gekennzeichnet, daß** sie in Form einer Lösung in einem biologisch kompatiblen Lösungsmittel vorliegt und daß sie auf subkutanem Weg, auf intramuskulärem Weg oder in einen Tumor injiziert werden kann.

17. Zusammensetzung nach Anspruch 16, **dadurch gekennzeichnet, daß** sie sich zur Bildung einer Ablagerung an der Injektionsstelle eignet.

18. Verfahren zur Herstellung:
• von Medikamenten für die orale, nasale, vaginale, okulare, subkutane, intravenöse, intramuskuläre, intradermale, intraperitoneale oder intrazerebrale Verabreichung, wobei es sich bei den Wirkstoffen dieser Medikamente um Proteine, Glykoproteine, an eine oder mehrere Polyalkylenglykolketten gebundene Proteine, Peptide, Polysaccharide, Liposaccharide, Oligonukleotide, Polynukleotide und organische Nichtproteinmoleküle, die hydrophob, hydrophil oder amphiphil sein können, handeln kann;
• und/oder von Nährmitteln;
• und/oder von kosmetischen Produkten oder Pflanzenschutzprodukten;
**dadurch gekennzeichnet, daß** es im wesentlichen daraus besteht, daß man mindestens eine Polyaminosäure nach einem der Ansprüche 1 bis 8 und/oder die Zusammensetzung nach einem der Ansprüche 9 bis 17 einsetzt.
